# EUROPÄISCHE PATENTSCHRIFT

(11) **EP 3 368 103 B1**
(45) Veröffentlichungstag und Bekanntmachung des Hinweises auf die Patenterteilung: **15.07.2020**
(21) Anmeldenummer: 16728325.8
(22) Anmeldetag: 10.06.2016
(51) Int. Cl.: A61M 5/158, A61M 39/24, A61M 5/168, A61M 39/26

(54) **VORRICHTUNG ZUR BEGRENZUNG DES INJEKTIONSDRUCKES EINES MEDIZINISCHEN INSTRUMENTS ZUM EINBRINGEN EINES FLUIDS**
DEVICE FOR LIMITING THE INJECTION PRESSURE OF A MEDICAL INSTRUMENT FOR INTRODUCING A FLUID
PROCÉDÉ POUR LIMITER LA PRESSION D'INJECTION D'UN INSTRUMENT MÉDICAL POUR INJECTER UN FLUIDE

(30) Priorität: 30.10.2015 US 201562285447 P
(43) Veröffentlichungstag der Anmeldung: 05.09.2018
(73) Patentinhaber: Pajunk GmbH Medizintechnologie, 78187 Geisingen (DE)
(72) Erfinder: CAPDEVILA, Xavier, 34980 Montferrier sur Lez (FR); CHOQUET, Olivier, 34170 Castelnau le Lez (FR); DESAI, Siddharth, Ladera Ranch, California 92694 (US)
(74) Vertreter: Westphal, Mussgnug & Partner Patentanwälte mbB
(86) Internationale Anmeldenummer: PCT/EP2016/063389
(87) Internationale Veröffentlichungsnummer: WO 2017/071833

(56) Entgegenhaltungen:
- US-A- 4 403 988
- US-A1- 2003 225 371
- US-A1- 2006 149 214
- US-A1- 2010 179 488

## Beschreibung

Die Erfindung betrifft eine Vorrichtung zur Begrenzung des Injektionsdruckes eines medizinischen Instruments zum Einbringen eines Fluids gemäß dem Oberbegriff des Patentanspruchs 1.

Als Injektion bezeichnet man in der Medizin das parenterale Einbringen eines Fluids, in der Regel einer Flüssigkeit. Das Fluid wird dabei relativ schnell verabreicht, wozu es mit einem gewissen Druck in den Körper eingeleitet wird. Der Druck kann dabei manuell, z. B. mittels eines Spritzenkolbens, oder auch mittels einer Pumpe erzeugt werden. Der Injektionsdruck, mit welchem das Fluid eingebracht wird, darf dabei in den meisten Fällen einen gewissen kritischen Druck nicht überschreiten, da anderenfalls Schädigungen verursacht werden können. Das Instrument zum Einbringen des Fluids ist in den meisten Fällen eine Kanüle. Die Erfindung ist jedoch auch bei anderen medizinischen Instrumenten verwendbar, die zum Einbringen eines Fluids dienen, z. B. bei Spritzen, Kathetern usw. Nachfolgend wird die Erfindung beispielhaft in Verbindung mit einer Kanüle beschrieben. Die Verwendung in Verbindung mit anderen medizinischen Instrumenten ergibt sich dabei in offensichtlicher entsprechender Weise.

Bei der peripheren Nervenblockade in der Anästhesie wird ein Fluid, d. h. ein Anästhetikum an den zu blockierenden Nerv injiziert. Dabei ist es wichtig, das Anästhetikum möglichst nahe an den Nerv zu applizieren, um eine effektive Anästhesie zu erreichen. Andererseits darf das Anästhetikum nicht in den Nerv injiziert werden, da dies unter Umständen schwerwiegende Schädigungen des Nervs zur Folge haben kann. Die Position der Kanüle bei der Injektion wird in der Regel durch elektrische Stimulation und/oder durch Ultraschallbeobachtung festgestellt. Zusätzlich kann die Position der Kanülenspitze dadurch überprüft werden, dass der Druck beobachtet wird, der sich aufgrund des unterschiedlichen Gewebewiderstands beim Injizieren in der Kanüle aufbaut. Das perineurale Gewebe bietet einen relativ geringen Widerstand, während der Widerstand deutlich ansteigt, wenn die Kanülenspitze auf das den Nerv umhüllende Epineurium trifft und insbesondere auf ein Nervenfaszikel. Der die Injektion durchführende Benutzer kann diesen Injektionsdruck spüren, da dieser dem Vorschieben des Spritzenstempels durch den Benutzer entgegenwirkt. Das Fühlen des Injektionsdrucks durch den Benutzer ist jedoch unzuverlässig. Daher wurde versucht, den Injektionsdruck der Kanüle objektiv zu ermitteln, um ein Injizieren des Anästhetikums in den Nerv zuverlässiger zu vermeiden.

Aus der US 4 403 988 ist es zu diesem Zweck bekannt, zwischen die Spritze und die Kanüle eine Vorrichtung einzusetzen, welche einen Durchflusskanal aufweist, der die Spritze mit der Kanüle verbindet. Von diesem Durchflusskanal zweigt seitlich ein Austrittskanal ab, der durch einen federbelasteten Kolben verschlossen ist. Trifft die Kanülenspitze bei der Injektion auf einen höheren Gewebewiderstand, weil die Kanülenspitze auf den Nerv trifft oder in diesen eindringt, so baut sich in der Kanüle und damit in dem Durchflusskanal der Vorrichtung ein höherer Injektionsdruck auf. Überschreitet dieser Injektionsdruck den durch die Federbelastung des Kolbens vorgegebenen Wert, so wird der Kolben gegen die Rückstellkraft der Feder aus seiner Schließstellung bewegt und gibt den Austrittskanal frei. Das Anästhetikum kann durch den Austrittskanal austreten, solange der Injektionsdruck über diesem vorgegebenen Wert liegt, so dass der Injektionsdruck abgebaut wird. Da der Injektionsdruck durch diese Vorrichtung auf den vorgegebenen Wert begrenzt wird, kann der die Injektion durchführende Benutzer kein Ansteigen des Injektionsdrucks über den vorgegebenen Wert feststellen, so dass er unter Umständen die falsche Position der Kanülenspitze nicht bemerkt und es zu einer Schädigung des Nervs kommen kann.

Aus der WO 03/101526 A1 ist eine Vorrichtung bekannt, die zwischen die Spitze und die Kanüle eingesetzt wird und einen die Spritze mit der Kanüle verbindenden Durchflusskanal aufweist. Der Durchflusskanal kommuniziert mit einer Druckkammer, welche durch eine Membran begrenzt ist. Steigt der Injektionsdruck in dem Durchflusskanal und damit in der Druckkammer an, so wird die Membran ausgelenkt und drückt einen Anzeigestift gegen die Kraft einer Feder, so dass dieser Anzeigestift entsprechend dem in dem Durchflusskanal herrschenden Injektionsdruck unterschiedlich weit aus dem Gehäuse der Vorrichtung herausgeschoben wird, wodurch über eine Farbringcodierung des Anzeigestiftes der Injektionsdruck visuell überwacht werden kann. Die Vorrichtung zeigt lediglich den Injektionsdruck an, begrenzt diesen jedoch nicht automatisch. Die Vermeidung einer schädigenden Injektion ist daher davon abhängig, wie schnell der die Injektion durchführende Benutzer die Anzeige des Druckanstiegs bemerkt und die sich daraus ergebenden Maßnahmen durchführt.

US 2006/149214 A1 und US 2003/225371 A1 bilden weiteren Stand der Technik.

Der Erfindung liegt die Aufgabe zugrunde, eine Vorrichtung zur Begrenzung des Injektionsdruckes zu schaffen, die eine Fehlinjektion zuverlässig und unabhängig von der die Injektion durchführenden Person verhindert.

Diese Aufgabe wird erfindungsgemäß gelöst durch eine Vorrichtung mit den Merkmalen des Patentanspruchs 1.

Vorteilhafte Ausführungen der Erfindung sind in den Unteransprüchen angegeben.

Die erfindungsgemäße Vorrichtung weist einen Durchflusskanal auf, durch welchen das zu injizierende Fluid, insbesondere z. B. ein Anästhetikum von einer durch die die Injektion durchführende Person betätigten Spritze zu einer in den Körper des Patienten einzustechenden Kanüle fließt. Der in der Kanüle und damit in dem Durchflusskanal herrschende Injektionsdruck beaufschlagt einen in dem Gehäuse der Vorrichtung gelagerten Kolben und kann diesen gegen eine Rückstellkraft bewegen, wenn der Injektionsdruck einen durch die Rückstellkraft vorgegebenen Wert überschreitet. An dem Kolben ist ein Verschlusselement angeordnet, welches mit dem Kolben bewegt wird. Das Verschlusselement ist in dem Strömungsquerschnitt des Durchflusskanals bewegbar. Liegt der Injektionsdruck unter dem vorgegebenen Wert, so gibt das Verschlusselement den Strömungsquerschnitt des Durchflusskanals frei, so dass das zu injizierende Fluid von der Spritze durch den Durchflusskanal zu der Kanüle strömen kann. Überschreitet der Injektionsdruck den vorgegebenen Wert, so dass der Kolben gegen die Rückstellkraft bewegt wird, so wird das Verschlusselement mittels des Kolbens in eine Sperrstellung bewegt, in welcher das Verschlusselement den Strömungsquerschnitt sperrt. Sobald der Injektionsdruck den vorgegebenen Wert überschreitet, sperrt die Vorrichtung somit automatisch den Durchfluss des Anästhetikums von der Spritze zu der Kanüle, so dass der Spritzendruck nicht mehr auf das Anästhetikum in der Kanüle einwirkt und kein Anästhetikum injiziert wird. Ein unbeabsichtigtes Injizieren des Anästhetikums in das Nervengewebe mit dem höheren Gewebewiderstand ist dadurch unabhängig von dem Benutzer und ohne zeitliche Verzögerung zuverlässig ausgeschlossen.

Der vorgegebene Wert des Injektionsdruckes, bei welchem die Vorrichtung sperrt, ist von verschiedenen Faktoren abhängig, insbesondere z. B. von den Dimensionen der Kanüle, d. h. von der Länge und dem Innendurchmesser der Kanüle, und von dem Patienten, z. B. von dem Alter des Patienten. In einer vorteilhaften Ausführung der Erfindung ist die auf den Kolben mit dem Verschlusselement wirkende Rückstellkraft verstellbar, so dass der vorgegebene Wert des Injektionsdruckes dem jeweiligen Anwendungsfall entsprechend eingestellt werden kann. Dies ist besonders dann von Vorteil, wenn die Vorrichtung ein separates Bauteil ist, welches zwischen die Spritze und die Kanüle eingesetzt wird. Ein einheitliches Bauteil kann somit für unterschiedliche Anwendungsfälle verwendet werden, wobei der zulässige Injektionsdruck entsprechend der verwendeten Kanüle und entsprechend dem Patienten gewählt und eingestellt werden kann.

In einer anderen Ausführung kann die erfindungsgemäße Vorrichtung in dem proximalen Ansatz der Kanüle integriert sein. Da die Kanülendimensionen in diesem Falle festliegen, kann hierbei auf eine Verstellbarkeit des zulässigen Injektionsdruckes verzichtet werden, so dass der Aufbau der Vorrichtung einfacher wird und die Benutzung der Vorrichtung keine zusätzlichen Maßnahmen erfordert.

In einer vorteilhaften Ausführung ist die Verstellbarkeit der auf den Kolben wirkenden Rückstellkraft und damit die Einstellung des zulässigen Injektionsdruckes in der Weise realisiert, dass die Rückstellkraft einerseits an dem Kolben und andererseits an einem Stützteil des Gehäuses angreift, wobei dieses Stützteil in dem Gehäuse axial verstellbar ist, um die Rückstellkraft einzustellen.

Die Rückstellkraft ist insbesondere eine Federkraft, die vorzugsweise durch eine Schraubendruckfeder bewirkt wird. Die Schraubendruckfeder ist koaxial zu dem Kolbenhubweg angeordnet und stützt sich einerseits an dem Kolben und andererseits an dem Stützteil des Gehäuses ab. In einer anderen Ausführung kann die Rückstellkraft eine Magnetkraft sein. Hierzu wird vorzugsweise die magnetische Abstoßkraft zweier Permanentmagnete verwendet, von denen einer an dem Kolben und der andere an dem Stützteil des Gehäuses angeordnet ist. Andere Möglichkeiten zur Erzeugung der Rückstellkraft stehen dem Fachmann zur Verfügung. Diese Möglichkeiten umfassen beispielsweise elastisch komprimierbare Elemente aus Gummi oder Schaumstoff sowie komprimierbare geschlossene Luft- bzw. Gasvolumina.

Der Eintritt und der Austritt des Durchflusskanals und damit der Vorrichtung liegen in einer Längsachsenlinie, so dass die eintrittsseitig konnektierte Spritze und die austrittsseitig konnektierte Kanüle axial fluchtend ausgerichtet sind. Des Weiteren ist der Kolben koaxial zu dem Durchflusskanal und der Längsachsenlinie angeordnet, wobei das Verschlusselement als axial mittels des Kolbens bewegbarer Ventilteller ausgebildet ist. Diese Ausführung hat den Vorteil einer schlanken Außenkontur der Vorrichtung.

In einem weiteren Beispiel, welches nicht Teil der Erfindung ist, ist der Kolben senkrecht zu der Längsachsenlinie und dem Durchflusskanal bewegbar. Das Verschlusselement ist zur Längsmittellinie senkrecht in dem Durchflusskanal bewegbar und verschließt in der Sperrstellung den spritzenseitigen Eintritt des Durchflusskanals.

Die Vorrichtung ist vorzugsweise als kostengünstiges Kunststoffteil ausgebildet, das auch als Einmal-Artikel verwendet werden kann.

Im Folgenden wird die Erfindung anhand von in der Zeichnung dargestellten Ausführungsbeispielen näher erläutert. Es zeigen:
Figur 1 in perspektivischer Ansicht eine Vorrichtung gemäß der Erfindung in einer ersten Ausführung,
Figur 2 einen Axialschnitt durch diese Vorrichtung,
Figur 3 eine Explosionsdarstellung der Vorrichtung,
Figur 4 einen Axialschnitt der Vorrichtung in der Durchflussstellung in der Einstellung mit niedrigem Injektionsdruck,
Figur 5 einen Figur 4 entsprechenden Axialschnitt der Vorrichtung in der Sperrstellung,
Figur 6 einen Figur 4 entsprechenden Axialschnitt der Vorrichtung in der Durchflussstellung in der Einstellung mit einem hohen Injektionsdruck,
Figur 7 einen Figur 6 entsprechenden Axialschnitt der Vorrichtung in der Sperrstellung,
Figur 8 eine Figur 1 entsprechende perspektivische Ansicht der Vorrichtung in der Einstellung mit hohem Injektionsdruck,
Figur 9 in perspektivischer Ansicht das Innenrohr der Vorrichtung in der ersten Ausführung,
Figur 10 in einer Seitenansicht das Innenrohr und die Verstellhülse in der Einstellung mit niedrigem Injektionsdruck,
Figur 11 einen Schnitt gemäß der Linie K-K in Figur 10,
Figur 12 eine Figur 10 entsprechende Darstellung in der Einstellung mit einem mittleren Injektionsdruck,
Figur 13 einen Schnitt gemäß der Linie L-L in Figur 12,
Figur 14 eine Figur 10 entsprechende Ansicht in der Einstellung mit hohem Injektionsdruck,
Figur 15 einen Axialschnitt der erfindungsgemäßen Vorrichtung in einer zweiten Ausführung,
Fig. 16 einen vergrößerten Teilausschnitt aus Figur 15,
Fig. 17 eine Explosionsdarstellung der Vorrichtung in der zweiten Ausführung,
Fig. 18 einen Axialschnitt der Vorrichtung in der zweiten Ausführung in der Durchflussstellung,
Fig. 19 einen Figur 18 entsprechenden Axialschnitt in der Sperrstellung,
Figur 20 eine perspektivische Ansicht eines weiteren Beispiels welches nicht Teil der Erfindung ist
Figur 21 einen Axialschnitt der Vorrichtung der Figur 20 in der Durchflussstellung,
Figur 22 einen Teilaxialschnitt gemäß Figur 21 in der Sperrstellung,
Figur 23 in perspektivischer Ansicht eine Vorrichtung gemäß der Erfindung in einer dritten Ausführung,
Figur 24 einen Axialschnitt durch diese Vorrichtung,
Figur 25 einen Axialschnitt der Vorrichtung in der Durchflussstellung in der Einstellung mit niedrigem Injektionsdruck,
Figur 26 einen Figur 25 entsprechenden Axialschnitt der Vorrichtung in der Sperrstellung,
Figur 27 einen Figur 25 entsprechenden Axialschnitt der Vorrichtung in der Durchflussstellung in der Einstellung mit einem hohen Injektionsdruck,
Figur 28 einen Figur 27 entsprechenden Axialschnitt der Vorrichtung in der Sperrstellung und
Figur 29 eine Figur 2 entsprechende Darstellung der erfindungsgemäßen Vorrichtung in einer vierten Ausführung.

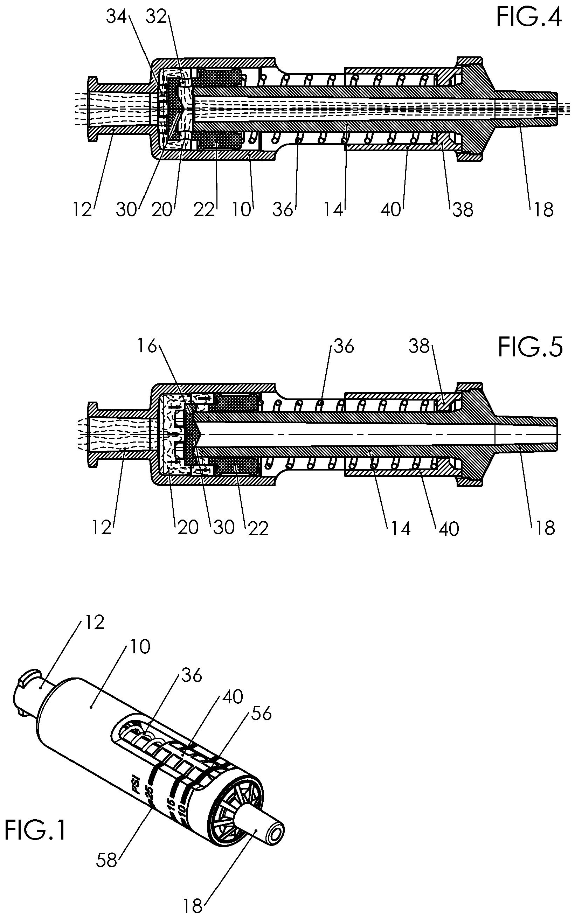

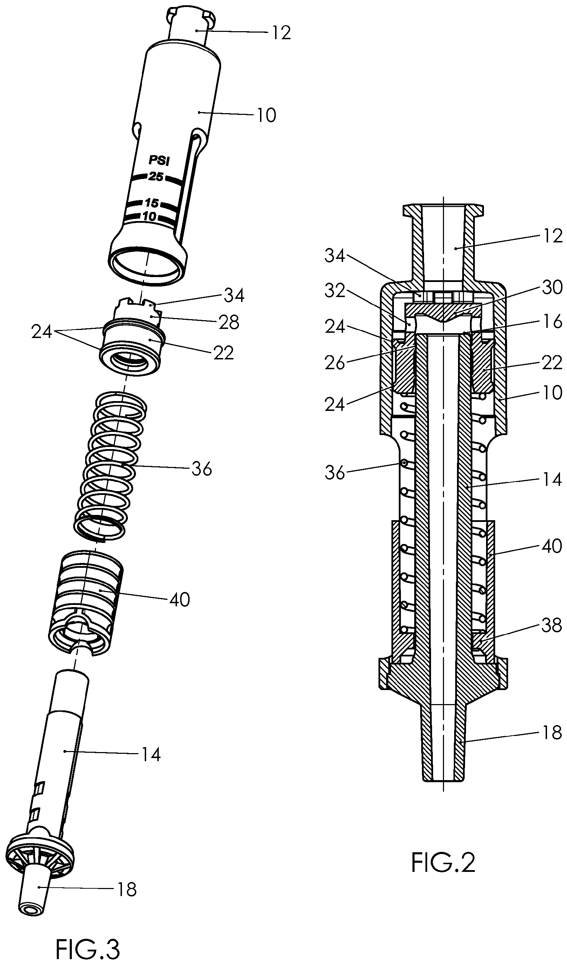

In der in den Figuren 1 bis 14 gezeigten ersten Ausführung weist die Vorrichtung ein Gehäuse 10 auf, welches im Wesentlichen die Form eines hohlen Kreiszylinders hat. Am proximalen Ende des Gehäuses 12 ist ein Eintritt 12 ausgebildet, der die Form eines Anschlusses aufweist, mit welchem ein nicht dargestellter Vorrat konnektierbar ist, von welchem ein Fluid unter Druck in das Gehäuse 10 eingeleitet werden kann. Ein solcher Vorrat ist vorzugsweise als Spritze ausgebildet, mit welcher ein Anästhetikum zugeführt wird. Der Anschluss des Eintritts 12 ist beispielsweise als weiblicher Luer-Lock-Anschluss ausgebildet.

In das distale Ende des Gehäuses 10 ist ein Innenrohr 14 eingesetzt. Das Innenrohr 14 verläuft koaxial in dem Gehäuse 10, wobei der Außendurchmesser des Innenrohrs 14 kleiner ist als der Innendurchmesser des Gehäuses 10, so dass ein zylindrischer Raum zwischen dem Innenrohr 14 und dem Gehäuse 10 gebildet wird. Das Innenrohr 14 ist an seinem stromaufwärts weisenden in dem Gehäuse 10 liegenden Ende offen und bildet einen kreisringförmigen Ventilsitz 16. Das distale stromabwärts liegende Ende des Innenrohrs 14 ragt aus dem Gehäuse 10 heraus und bildet einen Austritt 18, der als Anschluss ausgebildet ist, mit welchem eine nicht dargestellte Kanüle für die Injektion konnektierbar ist. Der Austritt 18 ist beispielsweise als männlicher Luer-Lock-Anschluss ausgebildet.

Der proximale Endbereich des Gehäuses 10 bildet einen Hubraum 20, in welchem ein Kolben 22 axial bewegbar ist. Der Kolben 22 ist axial bewegbar auf dem Innenrohr 14 geführt. An seinem Außenumfang ist der Kolben 22 mit Dichtlippen 24 gegenüber der Innenwandung des Gehäuses 10 abgedichtet. An seinem Innenumfang ist der Kolben 22 durch eine innere Dichtlippe 26 gegen den Außenumfang des Innenrohrs 14 abgedichtet.

An der dem Eintritt 12 zugewandten Stirnfläche des Kolbens 22 ist ein hohlzylindrischer Stutzen 28 angeformt, der durch einen Ventilteller 30 abgeschlossen ist. Der Ventilteller 30 hat die Form einer Kreisscheibe, deren Durchmesser den Außendurchmesser des Innenrohrs 14 überragt. Die Wandung des Stutzens 28 ist von Durchflussöffnungen 32 durchbrochen. An der stromaufwärts gegen den Eintritt 12 gerichteten Stirnfläche des Ventiltellers 30 sind über den Umfang verteilt und im Umfangswinkel gegeneinander beabstandete Distanzstege 34 angeformt.

Zur Erzeugung einer auf den Kolben 22 wirkenden Rückstellkraft wird das Innenrohr 14 koaxial von einer Schraubendruckfeder 36 umschlossen. Die Schraubendruckfeder 36 stützt sich mit ihrem stromauf gerichteten Ende an dem Kolben 22 ab. Das andere stromab gerichtete Ende der Schraubendruckfeder 36 stützt sich an einem Stützteil 38 ab, welches als Innenbund ausgebildet ist, der nach innen in das Gehäuse gegen das Innenrohr 14 gerichtet ist.

In einer alternativen Ausführung kann die Schraubendruckfeder 36 auch koaxial in das Innenrohr 14 eingesetzt sein. Dabei stützt sich die Schraubendruckfeder 36 mit ihrem stromaufgerichteten Ende an dem Ventilteller 30 des Kolbens 22 ab. Das stromabgerichtete Ende der Schraubendruckfeder 36 stützt sich an dem Stützteil 38 ab, welches in dieser Ausführung als Innenbund in dem Innenrohr 14 ausgebildet ist.

Anhand der Figuren 4 und 5 wird die Funktion der Vorrichtung erläutert. Zur Injektion eines Fluids, z. B. eines Anästhetikums für die periphere Nervenblockade, wird eine Spritze mit dem den Eintritt 12 bildenden Ansatz konnektiert und die Kanüle für den Einstich wird mit dem den Austritt 18 bildenden Ansatz konnektiert.

Zunächst drückt die Federkraft der Schraubendruckfeder 36 den Kolben 22 gegen die Einströmrichtung, d. h. in den Figuren 4 und 5 nach links. Die Bewegung des Kolbens 22 nach links wird dadurch begrenzt, dass die Distanzstege 34 an der eintrittsseitigen Stirnwand des Gehäuses 10 zum Anschlag kommen. Der Ventilteller 30 verschließt dadurch den Eintritt 12 nicht. Die Durchflussöffnungen 32 befinden sich dabei axial vor dem Ventilsitz 16 des Innenrohrs 14. Betätigt der die Injektion durchführende Benutzer die Spritze, so wird das Anästhetikum durch den Eintritt 12 eingeleitet, tritt zwischen den Distanzstegen 34 hindurch, umströmt den Ventilteller 30, tritt durch die Durchflussöffnungen 32 hindurch und gelangt in das Innenrohr 14, durch welches es zum Austritt 18 und damit in die Kanüle geleitet wird. Der Strömungsweg des Anästhetikums ist in den Figuren 4 und 5 durch gestrichelte Linien dargestellt. In dieser in Figur 4 gezeigten Durchflussstellung kann somit das Fluid ungehindert durch die Vorrichtung hindurchströmen und über die Kanüle injiziert werden.

Stößt die Kanülenspitze auf einen höheren Gewebewiderstand, z. B. beim Auftreffen auf das Epineurium oder beim Eindringen in den Nerv, so wird der Durchfluss durch die Kanüle erschwert und der von dem Benutzer auf die Spritze ausgeübte Druck führt zu einem ansteigenden Staudruck, der sich in der Kanüle, in dem Innenrohr 14 und in dem Innenraum des Gehäuses 10 stromauf vor dem Kolben 22 aufbaut. Wenn dieser Staudruck, der dem an der Kanülenspitze wirkenden Injektionsdruck entspricht, einen vorgegebenen Wert überschreitet, der durch die Federkraft der Schraubendruckfeder 36 vorgegeben ist, so drückt dieser Staudruck den Kolben 22 gegen die Federkraft der Schraubendruckfeder 36 nach rechts in die in Figur 5 gezeigte Stellung, in welcher die durch den Druck erzeugte Kraft durch Pfeile dargestellt ist. Wird der Kolben 22 auf dem Innenrohr 14 nach rechts in die in Figur 5 gezeigte Sperrstellung verschoben, so setzt sich der Ventilteller 30 auf den durch das freie innere Ende des Innenrohres 14 gebildeten Ventilsitz 16 und verschließt das Innenrohr 14. Die Durchflussöffnungen 32 werden dabei axial über das Innenrohr 14 geschoben, so dass auch diese Durchflussöffnungen 32 verschlossen sind. Dadurch ist der Durchfluss des Anästhetikums durch die Vorrichtung vollständig gesperrt und auf das Anästhetikum in der Kanüle wird kein Druck mehr ausgeübt.

Auch wenn der Benutzer den riskanten Einstich der Kanüle nicht bemerkt und weiter Druck auf den Spritzenstempel ausübt, ist eine schädliche Injektion zuverlässig ausgeschlossen. Wenn der Benutzer den Widerstand bei Betätigung der Spritze bemerkt und die Kanüle aus der Fehlpositionierung zurückzieht, kann die Schraubendruckfeder 36 den Kolben wieder in die Figur 4 gezeigte Durchflussstellung bewegen, so dass die Injektion nach der Korrektur der Kanülenposition fortgesetzt werden kann.

Der Injektionsdruck, bei welchem die Vorrichtung automatisch sperrt, ist durch die Federkraft der Schraubendruckfeder 36 vorgegeben. Der Injektionsdruck ist von verschiedenen Faktoren abhängig. Dies sind beispielsweise die Gewebebeschaffenheit des jeweiligen Patienten, der Innendurchmesser und die Länge der Kanüle und die Injektionsgeschwindigkeit. Es ist daher für den Benutzer von Vorteil, wenn er entsprechend diesen Faktoren den Injektionsdruck einstellen kann, bei welchem die Vorrichtung automatisch sperrt.

Dies wird in vorteilhafter Weise dadurch ermöglicht, dass die Vorspannung der Schraubendruckfeder 36 und damit die durch diese Schraubendruckfeder 36 erzeugte Rückstellkraft verstellbar ist.

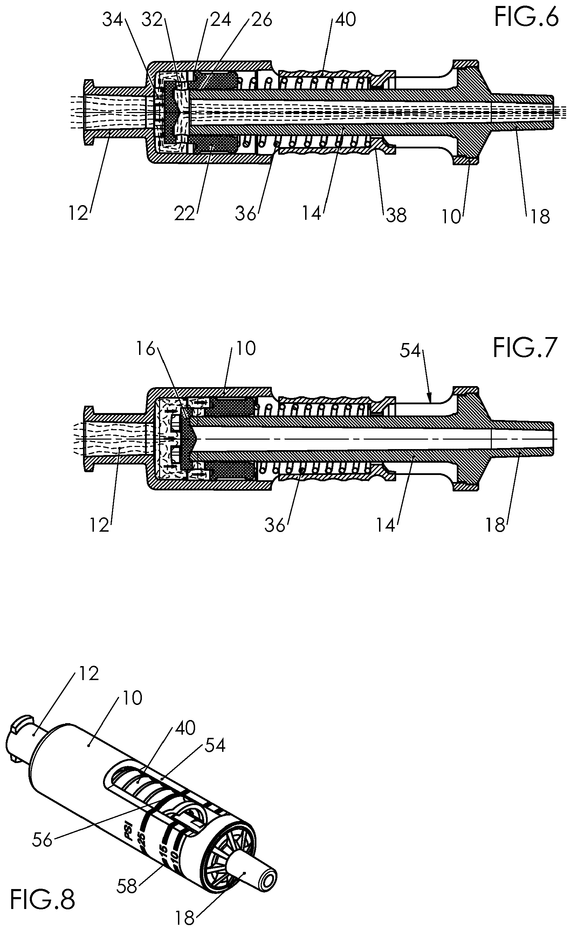

Hierzu ist vorzugsweise das Stützteil 38, auf welchem sich die Schraubendruckfeder 36 abstützt, in dem Gehäuse 10 in Axialrichtung der Schraubendruckfeder 36 verstellbar, so dass die Schraubendruckfeder 36 einstellbar vorgespannt werden kann. Das Stützteil 38 ist hierzu als Innenbund einer Verstellhülse 40 ausgebildet. Die Verstellhülse 40 umschließt koaxial das Innenrohr 14 und die auf dem Innenrohr 14 sitzende Schraubendruckfeder 36. Die Verstellhülse 40 ist koaxial in dem Gehäuse 10 bewegbar und kann in der gewünschten Axialposition fixiert werden. Die Figuren 6, 7 und 8 zeigen in den Figuren 1, 4 und 5 entsprechenden Darstellungen die Vorrichtung mit einer die Schraubendruckfeder 36 vorspannenden Einstellung der Verstellhülse 40, wodurch ein Sperren des Durchflusses erst bei einem höheren Injektionsdruck bewirkt wird.

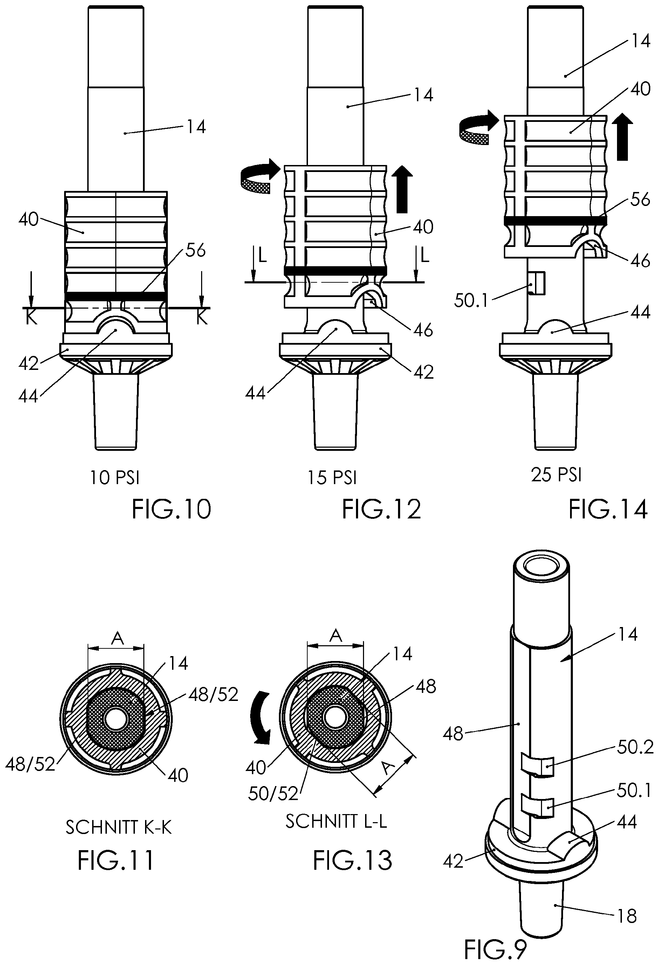

Der Aufbau der Verstellung der Federkraft ist im Einzelnen aus den Figuren 9 bis 14 zu erkennen. Es sind hier beispielhaft drei Einstellungswerte des zulässigen Injektionsdruckes dargestellt, nämlich 10 psi, 15 psi und 25 psi (0,7 bar, 1,0 bar und 1,7 bar). Bei der Einstellung mit dem niedrigsten die Sperrfunktion auslösenden Injektionsdruck von 10 psi (Figuren 10 und 11) sitzt die Verstellhülse 40 axial auf einem Flansch 42 auf, mit welchem das Innenrohr 14 in dem Gehäuse 10 befestigt ist. Eine Nase 44 des Flansches 42 greift in eine Aussparung 46 der Verstellhülse 40 und hält diese verdrehfest auf dem Innenrohr 14. Das Innenrohr 14 weist an seiner äußeren Mantelfläche zwei planparallele Abflachungen 48 auf, die einen diametralen Abstand A aufweisen, der kleiner ist als der Außendurchmesser des Innenrohr 14. Weiter weist das Innenrohr 14 zwei von dem Flansch 42 axial unterschiedlich beabstandete Paare von Einkerbungen 50.1 und 50.2 auf. Das Paar der Einkerbungen 50.1 weist planparallele Flächen auf, die in einem diametralen Abstand A angeordnet sind und in Umfangsrichtung um 45° gegenüber den Abflachungen 48 versetzt angeordnet sind. Das Paar der Einkerbungen 50.2 entspricht in der Ausführung und Position den Einkerbungen 50.1 ist jedoch axial gegenüber den Einkerbungen 50.1 versetzt. Die Verstellhülse 40 weist einen kreisrunden Innendurchmesser auf, der dem kreisrunden Außendurchmesser des Innenrohres 14 entspricht. Der kreisrunde Innendurchmesser ist in einem Bereich des dem Flansch 42 zugewandten Endes der Verstellhülse 40 mit zwei planparallelen diametral zueinander angeordneten Sekantenflächen 52 verengt, die einen lichten Abstand A aufweisen.

Wie aus den Figuren 10 bis 14 hervorgeht, erfolgt die axiale Verstellung der Verstellhülse 40 und damit die Einstellung der Vorspannung der Schraubendruckfeder 36 in folgender Weise. In der Einstellung mit der kleinsten Federvorspannung, die in den Figuren 10 und 11 gezeigt ist, befindet sich die Verstellhülse 40 in einer solchen Drehstellung auf dem Innenrohr 14, dass die vom Innenumfang des Innenrohrs 14 nach innen vorspringenden Sekantenflächen 52 mit den Abflachungen 48 des Innenrohrs 40 zusammen fallen. Die Verstellhülse 40 kann sich daher auf dem Innenrohr 14 nach unten (in der Darstellung der Figuren 10 bis 14), d. h. gegen den Flansch 42 bewegen, bis die Verstellhülse 40 auf dem Flansch 42 aufsitzt und die Nase 44 in die Aussparung 48 eingreift, wie dies in den Figuren 10 und 11 dargestellt ist. Bei dieser geringsten Federvorspannung und damit geringster auf den Kolben 22 wirkender Federkraft sperrt die Vorrichtung bei einem Injektionsdruck von beispielsweise 10 psi.

Die Verstellhülse 40 kann aus dieser Stellung axial von dem Flansch 42 abgehoben werden, wobei die Sekantenflächen 52 der Verstellhülse 40 auf den Abflachungen 48 des Innenrohres 14 gleiten. Wenn die nach innen vorspringenden Sekantenflächen 52 axial mit den Einkerbungen 50.1 zur Deckung kommen, kann die Verstellhülse 40 um das Innenrohr 14 gedreht werden, wie dies in den Figuren 12 und 13 gezeigt ist, wobei die Sekantenflächen 52 in die Einkerbungen 50.1 eingreifen, wie dies aus Figur 13 ersichtlich ist. Dadurch wird die Verstellhülse 40 in der den Einkerbungen 50.1 entsprechenden axialen Position auf dem Innenrohr 14 arretiert. Dadurch wird die Schraubendruckfeder 36 entsprechend vorgespannt und die auf den Kolben 22 wirkende Federkraft erhöht, so dass die Vorrichtung erst bei dem höheren Injektionsdruck von z. B. 15 psi sperrt. Wird die Verstellhülse 40 wieder in die Winkelstellung gedreht, in welcher die Sekantenflächen 52 mit den Abflachungen 48 zusammenfallen, so kommen die Sekantenflächen 52 aus den Einkerbungen 50.1 frei und die Verstellhülse 40 kann in axialer Richtung weiter nach oben von dem Flansch 42 abgehoben werden, wie dies Figur 14 zeigt. Sobald die Sekantenflächen 52 mit dem nächsten Paar der Einkerbungen 50.2 axial zur Deckung kommen, kann die Verstellhülse 40 wieder um 45° gedreht werden, entsprechend der Darstellung der Figur 13, so dass die Verstellhülse 40 wieder in entsprechender Weise in den Einkerbungen 50.2 arretiert wird. Die Schraubendruckfeder 36 wird hierdurch entsprechend stärker vorgespannt, wodurch die auf den Kolben 22 wirkende Federkraft weiter erhöht wird. Dies entspricht einer Sperrung des Durchflusses bei einem entsprechend höheren Injektionsdruck von z. B. 25 psi.

Zum Verstellen der Verstellhülse 40 weist das Gehäuse 10 in seiner Mantelfläche zwei diametral angeordnete Fenster 54 auf, durch welche die Verstellhülse 40 mit den Fingern erfasst und verschoben und verdreht werden kann. Die jeweilige Verstellposition der Verstellhülse 40 und damit der jeweils eingestellte Injektionsdruck wird durch eine Markierung 56 auf dem Außenumfang der Verstellhülse 40 und eine Skala 58 an den Fenstern 54 des Gehäuses 10 angezeigt.

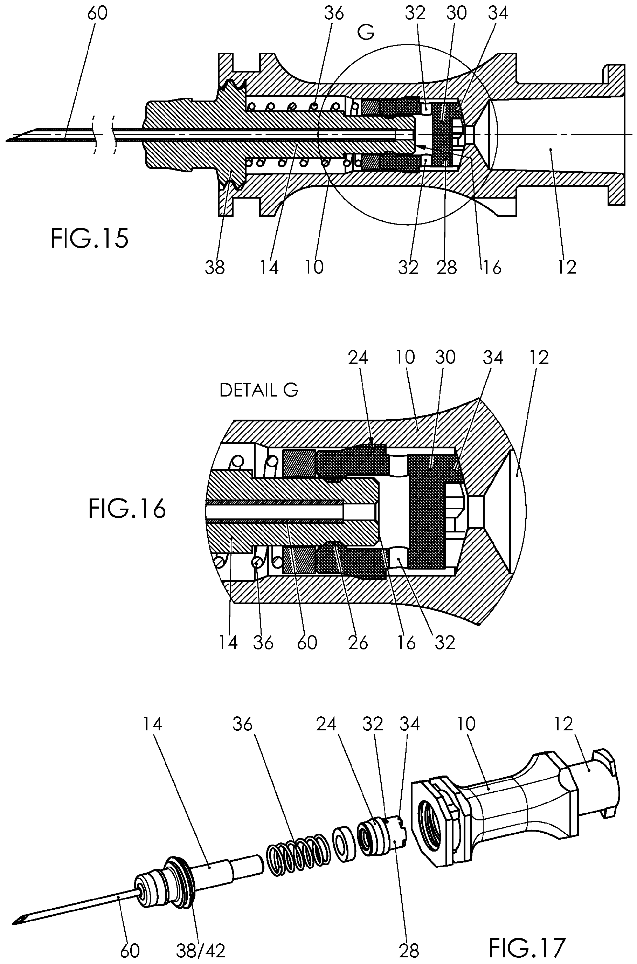

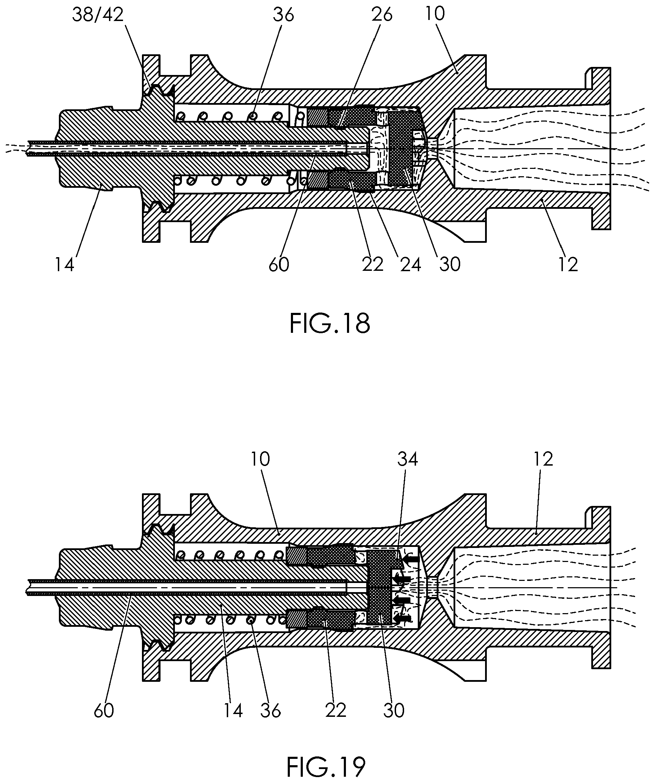

In den Figuren 15 bis 19 ist eine zweite Ausführung der erfindungsgemäßen Vorrichtung dargestellt.

Diese Ausführung stimmt in der wesentlichen Funktion mit dem ersten Ausführungsbeispiel überein. Insoweit werden dieselben Bezugszeichen verwendet und auf die vorhergehende Beschreibung wird Bezug genommen. Der wesentliche Unterschied gegenüber dem ersten Ausführungsbeispiel besteht darin, dass in dieser Ausführung die Vorrichtung zur Begrenzung des Injektionsdruckes in den proximalen Ansatz einer Kanüle integriert ist, so dass es sich nicht um ein gesondertes Bauteil handelt. Da die Vorrichtung zur Begrenzung des Injektionsdruckes in den Kanülenansatz integriert ist, ist die Vorrichtung dadurch einer bestimmten Kanüle mit bestimmten vorgegebenen Dimensionen zugeordnet. Es erübrigt sich daher in der Regel eine Verstellbarkeit der auf den Kolben wirkenden Federkraft zur Anpassung an den Injektionsdruck.

Da in dieser Ausführung keine Verstellung der den Kolben beaufschlagenden Federkraft vorgesehen ist, entfällt eine Verstellbarkeit des Stützteils, an welchem die Schraubendruckfeder 36 abgestützt ist. Das Stützteil wird durch den Flansch 42 gebildet, mit welchem das Innenrohr 14 in das Gehäuse 10 eingesetzt ist. Weiter kann eine einfache Integration in der Weise erfolgen, dass das proximale Ende des Kanülenrohres 60 der mit der Vorrichtung versehenen Kanüle koaxial in das Gehäuse 10 hineinführt und in das Innenrohr 14 eingespritzt ist. Dadurch ist die Vorrichtung erheblich vereinfacht und besonders kostengünstig herstellbar, was die Integration mit der Einmal-Kanüle begünstigt.

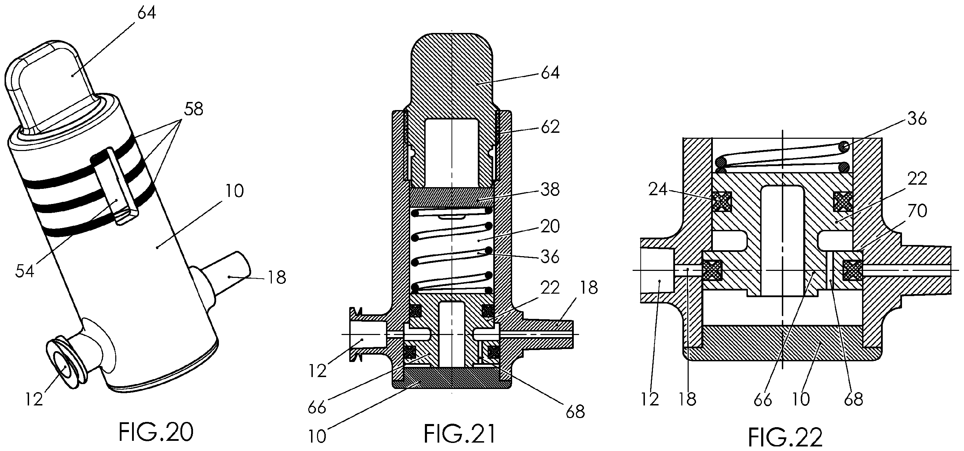

In den Figuren 20 bis 22 ist ein weiteres Beispiel gezeigt, welches nicht Teil der Erfindung ist.

In dieser Ausführung weist das Gehäuse 10 einen Eintritt 12 und einen axial mit diesem Eintritt 12 fluchtenden Austritt 18 auf. Der Eintritt 12 und der Austritt 18 sind jeweils als Anschlüsse zum Konnektieren einer Spritze bzw. einer Kanüle ausgebildet. Senkrecht zu der durch den Eintritt 12 und den Austritt 18 definierten Längsachsenlinie bildet das Gehäuse 10 einen Hubraum 20, in welchem ein Kolben 22 gelagert ist, der in der zur Längsmittelachse senkrechten Achse des Hubraums 20 bewegbar ist. Der Kolben 22 ist gegen die Innenwand des Gehäuses 10 mittels einer Dichtlippe 24 abgedichtet. Das Gehäuse 10 bildet einen von dem Eintritt 12 zu dem Austritt 18 führenden Durchlasskanal, der seitlich durch den Kolben 22 abgeschlossen ist. Auf den Kolben 22 wirkt eine Federkraft, die durch eine Schraubendruckfeder 36 erzeugt wird. Die Schraubendruckfeder 36 stützt sich einerseits an dem Kolben 22 ab und andererseits an einem Stützteil 38. Das Stützteil 38 ist in das radial nach außen ragende Ende des Gehäuses 10 eingesetzt und mittels eines selbsthemmenden Gewindes 62 axial in der Hubrichtung des Kolbens 22 verstellbar. Zur Verstellung dient ein aus dem Gehäuse 10 herausragenden Griff 64. Im Bereich des Verstellweges des Stützteils 38 sind in der Wandung des Gehäuses 10 Fenster 54 vorgesehen, durch welche eine Markierung 56 des Stützteils 38 sichtbar ist und die axiale Stellung des Stützteils 38 mittels einer außen an den Fenstern 54 vorgesehenen Skala 58 abgelesen werden kann. Durch Verdrehen des Stützteils 38 kann über das Gewinde 62 die axiale Position des Stützteils 38 und damit die Vorspannung der sich an dem Stützteil 38 abstützenden Schraubendruckfeder 36 eingestellt werden.

An der dem Durchflusskanal zugewandten Stirnfläche des Kolbens 22 ist ein als Verschlusselement dienender Ventilschieber 66 angebracht. Der Ventilschieber hat die Funktion, den Eintritt 12 in das Gehäuse je nach Stellung des Ventilschiebers 66 freizugeben, um den Durchfluss zu dem Austritt 18 zu ermöglichen, oder den Eintritt 12 zu verschließen, um den Durchfluss zu sperren. Der Ventilschieber 66 hat im dargestellten Ausführungsbeispiel die Form einer Kreisscheibe, die axial von der Stirnfläche des Kolbens 22 beabstandet ist, im Außendurchmesser dem Innendurchmesser des Hubraums 20 entspricht und deren axiale Dicke größer als der Innendurchmesser des Eintritts 12 ist. Der Ventilschieber 66 kann mittels des Kolbens 22 zwischen einer in Figur 21 gezeigten Durchflussstellung und einer in Figur 22 gezeigten Sperrstellung bewegt werden. In der Durchflussstellung befindet sich der Ventilschieber 66 unterhalb des von dem Eintritt 12 zu dem Austritt 18 führenden Durchflusskanal, so dass das durch den Eintritt 12 eingeleitete Fluid zwischen dem Ventilschieber 66 und dem Kolben 22 hindurch zu dem Austritt 18 fließen kann. In der Sperrstellung (Figur 22) wird der Ventilschieber 66 mittels des Kolbens 22 angehoben, so dass er den Eintritt 12 verschließt, wodurch der Durchfluss von dem Eintritt 12 zu dem Austritt 18 gesperrt ist.

Die Funktionsweise der Vorrichtung in diesem Beispiel entspricht der Funktionsweise, die in Verbindung mit dem ersten Ausführungsbeispiel beschrieben ist, so dass hierauf Bezug genommen wird.

Solange der Injektionsdruck, der sich in der Kanüle und dem Durchflusskanal aufbaut unter dem durch die Federkraft auf den Kolben 22 wirkenden Druck liegt, hält die Schraubendruckfeder 36 den Kolben in der in Figur 21 gezeigten unteren Stellung, in welcher der Ventilschieber 66 den Durchlass von dem Eintritt 12 zu dem Austritt 18 und damit von der Spritze zu der Kanüle freigibt. Steigt der Injektionsdruck über den durch die Vorspannung der Schraubendruckfeder 36 eingestellten Wert, so baut sich dieser Injektionsdruck in dem Durchflusskanal zwischen dem Kolben 22 und dem Ventilschieber 66 auf. Dieser Staudruck wirkt über Durchlassöffnungen 68 des Ventilschiebers 66 auch in dem Raum unter dem Ventilschieber 66. Dieser den vorgegebenen Wert überschreitende Staudruck drückt den Kolben 22 und den Ventilschieber 66 gegen die Kraft der Schraubenfeder 36 nach oben (in der Darstellung der Figuren 21 und 22), so dass der Ventilschieber 66 in die in Figur 22 gezeigte Sperrstellung gelangt, in welcher er den Eintritt 12 verschließt und somit den Zufluss des Anästhetikums zu der Kanüle sperrt. In dieser Sperrstellung kommt der Ventilschieber 66 zur Anlage an einem Anschlag 70, so dass er durch den Staudruck nicht über die Sperrstellung hinaus gedrückt werden kann.

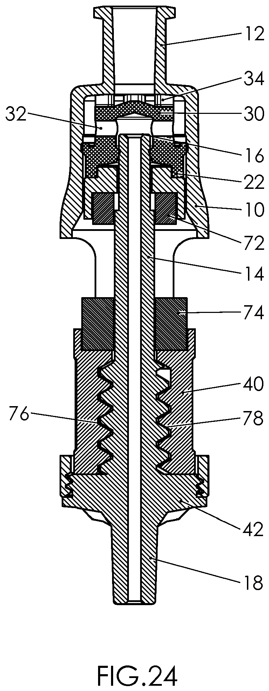

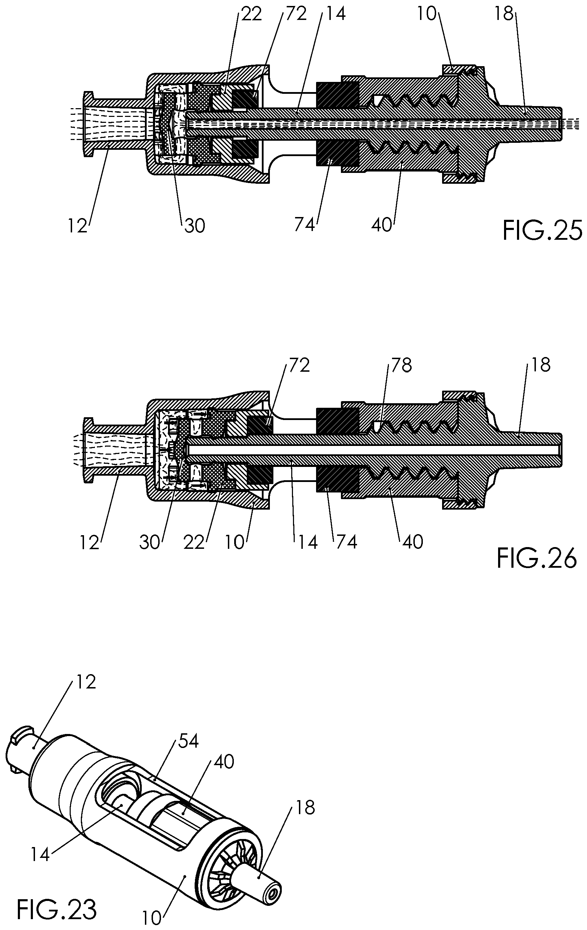

In den Figuren 23 bis 28 ist eine dritte Ausführung der Vorrichtung dargestellt. Die Vorrichtung in dieser Ausführung entspricht im Wesentlichen der Ausführung der Figuren 1 bis 8. Soweit Übereinstimmung mit dieser Ausführung der Figuren 1 bis 8 besteht, werden daher dieselben Bezugszahlen verwendet und auf die vorangehende Beschreibung wird Bezug genommen.

Die Ausführung der Figuren 23 bis 28 unterscheidet sich im Wesentlichen von der Ausführung der Figuren 1 bis 8 darin, dass die auf den Kolben 22 wirkende Rückstellkraft nicht als Federkraft z. B. einer Schraubendruckfeder erzeugt wird, sondern durch eine Magnetkraft. Eine solche magnetische Rückstellkraft ist in den Figuren 23 bis 28 in einer Ausführung beschrieben, die der Ausführung der Figuren 1 bis 8 entspricht. Es ist ohne weiteres ersichtlich, dass eine magnetische Rückstellkraft anstelle der Rückstellkraft eine Schraubendruckfeder auch bei der Vorrichtung in der Ausführung der Figuren 15 bis 19 und in der Ausführung der Figuren 20 bis 22 möglich ist.

In der dritten Ausführung entsprechen das Innenrohr 14, der Ventilsitz 16, der Kolben 22, der Stutzen 28, der Ventilteller 30, die Durchflussöffnungen 32 und die Distanzstege 34 der ersten Ausführungsform, wie sie im Zusammenhang mit dieser ersten Ausführungsform im Einzelnen beschrieben sind.

Zur Erzeugung der auf dem Kolben 22 wirkenden Rückstellkraft dienen zwei Magnete 72 und 74, die insbesondere als Permanentmagnete ausgebildet sind. Der eine Magnet 72 ist an der stromabgerichteten Stirnfläche des Kolbens 22 angebracht. Der andere Magnet 74 ist an der stromaufgerichteten Stirnfläche des Stutzteils 38 angebracht. Die Magnete 72 und 74 sind so gepolt, dass sie sich gegenseitig abstoßen. Der Magnet 72 ist zusammen mit dem Kolben 22 auf dem Innenrohr 14 axial geführt verschiebbar. Der andere Magnet 74 kann fest an einem durch einen Innenbund des Gehäuses 10 gebildeten Stützteil angebracht sein. In diesem Falle hat die auf dem Kolben 22 wirkende Magnetkraft einen vorgegebenen festen Wert, der den Injektionsdruck begrenzt.

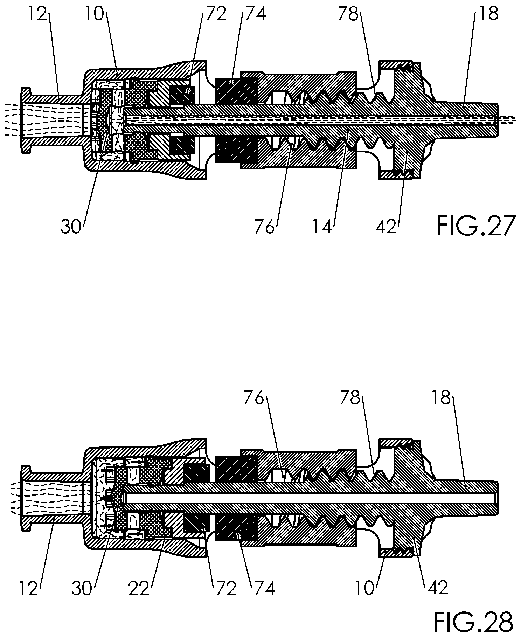

Soll die Rückstellkraft und damit der den Injektionsdruck begrenzende Wert verstellbar sein, so ist das Stützteil 38 axial in dem Gehäuse verstellbar, wie dies in der Ausführung der Figuren 23 bis 28 dargestellt ist. Das Stützteil 38, an welchem der Magnet 74 angebracht ist, ist in dieser Ausführung als Verstellhülse 40 ausgebildet, welche mit einem Innengewinde 76 in ein Außengewinde 78 des Innenrohres 14 eingreift. In der in den Figuren 25 und 26 gezeigten Einstellung mit niedrigem Injektionsdruck ist die Verstellhülse 40 auf dem Innenrohr 14 stromab, d. h. in der Zeichnung nach rechts gedreht, bis die Verstellhülse 40 an dem Flansch 42 anschlägt, mit welchem das Innenrohr 14 in das Gehäuse 10 eingeschraubt ist. Die Magnete 72 und 74 weisen in dieser Stellung den maximalen gegenseitigen Abstand auf, sodass die auf dem Kolben 22 wirkende Magnetkraft klein ist. Wird die Verstellhülse 40 mittels des Gewindes 76, 78 auf dem Innenrohr 14 stromauf, d. h. in der Zeichnung nach links verdreht, so verringert sich der axiale Abstand zwischen dem an dem Kolben 22 angebrachten Magnet 72 und dem an der Verstellhülse 40 angebrachten Magnet 74, sodass die magnetische Abstoßungskraft zwischen den Magneten 72 und 74 und damit die auf den Kolben 22 wirkende Rückstellkraft zunimmt. Diese Stellung ist in den Figuren 27 und 28 gezeigt. Entsprechend der höheren Rückstellkraft sperrt die Vorrichtung bei einem höheren Druck des Fluids in den Durchflusskanal.

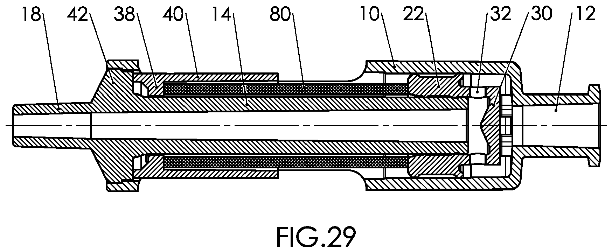

In Figur 29 ist eine weitere abgewandelte Ausführungsform gezeigt, die im Wesentlichen der ersten Ausführung entspricht. Es sind daher dieselben Bezugszahlen verwendet und auf die detaillierte Beschreibung der ersten Ausführung wird Bezug genommen. Die fünfte Ausführung unterscheidet sich von der ersten Ausführung darin, dass zur Erzeugung der auf den Kolben 22 wirkenden Rückstellkraft anstelle der Schraubendruckfeder 36 ein elastisch komprimierbares Element 80 eingesetzt ist, welches das Innenrohr 14 koaxial umschließt und sich einerseits an dem Kolben 22 und andererseits an dem Stützteil 38 abstützt. Das elastisch komprimierbare Element 80 kann beispielsweise aus einem gummielastischen Material oder einem elastisch komprimierbaren Schaumstoff bestehen. Ebenso ist es möglich, als komprimierbares Element 80 ein abgeschlossenes Luft- bzw. Gasvolumen einzusetzen. Wesentlich ist nur, dass das elastisch komprimierbare Element 80 eine von der axialen Kompression abhängige Rückstellkraft erzeugt. Selbstverständlich kann ein solches elastisch komprimierbares Element 80 auch bei den Ausführungsformen der Figuren 15 - 19 und 20 - 22 anstelle der Schraubendruckfeder 36 verwendet werden.

**Bezugszeichenliste**

| | |
|---|---|
| 10 | Gehäuse |
| 12 | Eintritt |
| 14 | Innenrohr |
| 16 | Ventilsitz |
| 18 | Austritt |
| 20 | Hubraum |
| 22 | Kolben |
| 24 | äußere Dichtlippen |
| 26 | innere Dichtlippen |
| 28 | Stutzen |
| 30 | Ventilteller |
| 32 | Durchflussöffnungen |
| 34 | Distanzstege |
| 36 | Schraubendruckfeder |
| 38 | Stützteil |
| 40 | Verstellhülse |
| 42 | Flansch |
| 44 | Nase |
| 46 | Aussparung |
| 48 | Abflachungen |
| 50 | Einkerbungen |
| 52 | Sekantenflächen |
| 54 | Fenster |
| 56 | Markierung |
| 58 | Skala |
| 60 | Kanülenrohr |
| 62 | Gewinde |
| 64 | Griff |
| 66 | Ventilschieber |
| 68 | Durchlassöffnung |
| 70 | Anschlag |
| 72 | Magnet |
| 74 | Magnet |
| 76 | Innengewinde |
| 78 | Außengewinde |
| 80 | komprimierbares Element |

## Patentansprüche

1. Vorrichtung zur Begrenzung des Injektionsdruckes eines medizinischen Instruments zum Einbringen eines Fluids, mit einem Gehäuse (10), mit einem Eintritt (12) des Gehäuses (10) zum Einleiten des Fluids unter einem Eintrittsdruck, mit einem Austritt (18) des Gehäuses (10) zum Zuführen des Fluids zu dem Instrument, mit einem den Eintritt (12) und den Austritt (18) verbindenden Durchflusskanal des Gehäuses (10) und mit einem in dem Gehäuse (10) geführten Kolben (22), der durch den Druck des Fluids in dem Durchflusskanal beaufschlagt ist und durch diesen Druck gegen eine Rückstellkraft bewegbar ist,
wobei das Gehäuse (10) einen Hubraum (20) aufweist, in welchem der Kolben (22) abgedichtet axial geführt ist, wobei die Rückstellkraft zur Kolbenbewegungsrichtung koaxial wirkt und einerseits an dem Kolben (22) und andererseits an einem Stützteil (38) des Gehäuses (10) angreift, wobei an dem Kolben (22) ein Verschlusselement (30, 66) angeordnet ist, welches sich in dem Strömungsquerschnitt des Duchflusskanals befindet,
wobei das Verschlusselement (30, 66) den Strömungsquerschnitt freigibt, wenn der den Kolben (22) beaufschlagende Druck des Fluids unter einem durch die Rückstellkraft vorgegebenen Wert liegt, und den Strömungsquerschnitt sperrt, wenn der den Kolben (22) beaufschlagende Druck des Fluids diesen vorgegebenen Wert überschreitet und den Kolben (22) gegen die Rückstellkraft bewegt,
wobei der Eintritt (12) und der Austritt (18) in einer gemeinsamen Längsachsenlinie angeordnet sind und der Hubraum (20) in dem Gehäuse (10) koaxial zu dieser Längsachsenlinie ausgebildet ist, **dadurch gekennzeichnet, dass** der Austritt (18) als koaxial in das Gehäuse (10) ragendes Innenrohr (14) ausgebildet ist, welches mit einem strom-aufwärts liegenden offenen Ende in den Hubraum (20) mündet, wobei der Kolben (22) auf diesem Innenrohr (14) geführt ist und das Verschlusselement ein Ventilteller (30) ist, der axial an dem Kolben (22) angebracht ist, der dichtend axial auf dem offenen Ende (16) des Innenrohres (14) aufsitzt, um den Strömungsquerschnitt zu sperren, und der von dem offenen Ende (16) des Innenrohres (14) axial abgehoben ist, um den Strömungsquerschnitt freizugeben, wobei an der dem Eintritt (12) zugewandten Stirnfläche des Kolbens (22) ein hohlzylindrischer Stutzen (28) angeformt ist, der durch den Ventilteller (30) abgeschlossen ist,
wobei der Ventilteller (30) die Form einer Kreisscheibe hat, deren Durchmesser den Außendurchmesser des Innenrohrs (14) überragt und wobei eine Wandung des Stutzens (28) von Durchflussöffnungen (32) durchbrochen ist.

2. Vorrichtung nach Anspruch 1,
**dadurch gekennzeichnet, dass** die Rückstellkraft verstellbar ist.

3. Vorrichtung nach den Ansprüchen 1 und 2,
**dadurch gekennzeichnet, dass** zur Verstellung der Rückstellkraft das Stützteil (38) in dem Gehäuse (10) axial verstellbar ist.

4. Vorrichtung nach einem der Ansprüche 1 bis 3,
**dadurch gekennzeichnet, dass** die Rückstellkraft durch eine zur Kolbenbewegungsrichtung koaxiale Schraubendruckfeder (36) bewirkt wird, die einerseits an dem Kolben (22) und andererseits an dem Stützteil (38) des Gehäuses (10) abgestützt ist.

5. Vorrichtung nach einem der Ansprüche 1 bis 4,
**dadurch gekennzeichnet, dass** die Rückstellkraft durch ein in Kolbenbewegungsrichtung elastisch komprimierbares Element (80) bewirkt wird, das einerseits an dem Kolben (22) und andererseits an dem Stützteil (38) des Gehäuses (10) abgestützt ist.

6. Vorrichtung nach einem der Ansprüche 1 bis 5,
**dadurch gekennzeichnet, dass** die Rückstellkraft durch sich abstoßende Permanentmagnete bewirkt wird, von denen einer an dem Kolben (22) und der andere an dem Stützteil (38) des Gehäuses (10) angeordnet ist.

7. Vorrichtung nach einem der Ansprüche 1 bis 6,
**dadurch gekennzeichnet, dass** die Vorrichtung ein separates Bauteil ist, das mit einem den Eintritt (12) bildenden Anschluss und mit einem den Austritt (18) bildenden Anschluss konnektierbar ist.

8. Vorrichtung nach einem der Ansprüche 1 bis 7,
**dadurch gekennzeichnet, dass** die Vorrichtung in einen proximalen Ansatz einer Kanüle integriert ist.

## Claims

1. A device for limitation of the injection pressure of a medical instrument for introduction of a fluid, having a housing (10), with an entrance (12) of the housing (10) for introducing the fluid at an entry pressure, with an outlet (18) of the housing (10) for introducing the fluid to the instrument, with a flow-through channel of the housing (10) connecting the entrance (12) and the outlet (18) and with a plunger (22) guided in the housing (10) which is impinged on by the pressure of the fluid and through this pressure is movable against a resetting force,
wherein the housing (10) has a stroke space (20), in which the plunger (22) is axially guided in sealed fashion, and wherein the resetting force acts coaxially to the plunger motion direction and engages on the one side on the plunger (22) and on the other side on a support piece (38) of the housing (10), wherein on the plunger (22) a blocking element (30, 66) is arranged, which is located in the flow cross section of the flow-through channel, and wherein the blocking element (30, 66) releases the flow cross section, when the pressure of the fluid impinging on the plunger (22) lies beneath a value preset by the resetting force, and blocks the flow cross section when the pressure of the fluid impinging on the plunger (22) exceeds this preset value and moves the plunger (22) against the resetting force,
wherein the entrance (12) and the outlet (18) are arranged on a common longitudinal axis line and the stroke space (20) is configured in the housing (10) to be coaxial to the longitudinal axis line of same,
**characterized in that** the outlet (18) is configured as an interior tube (14) projecting coaxially into the housing (10), which empties into the stroke space (20) with one open end lying upstream, wherein the plunger (22) is guided on this interior tube (14) and the blocking element is a valve disk (30) that is axially attached to the plunger (22), which sits in sealing fashion axially on the open end (16) of the interior tube (14), to block the flow cross section, and which is axially displaced from the open end (16) of the interior tube (14) to release the flow cross section,
wherein on the front side of plunger 22 facing entrance 12, a hollow cylindrical stub 28 is shaped, which is closed by the valve disk 30, wherein the valve disk 30 has the shape of a circular disk, the diameter of which projects over the outer diameter of interior tube 14 and wherein the wall of stub 28 is interrupted by flow-through openings 32.

2. The device of claim 1,
**characterized in that** the resetting force is adjustable.

3. The device of claims 1 and 2,
**characterized in that** for adjustment of the resetting force, the support piece (38) is axially adjustable in the housing (10) .

4. The device of one of claims 1 to 3,
**characterized in that** the resetting force is affected by a helical compression spring (36) coaxial to the direction of plunger motion, which is braced on the one side on the plunger (22) and on the other side on the support piece (38) of the housing (10).

5. The device of one of claims 1 to 4,
**characterized in that** the resetting force is affected by an elastically compressible element (80) in the direction of plunger motion, which is braced on the one side on the plunger (22) and on the other side on the support piece (38) of the housing (10).

6. The device of one of claims 1 to 5,
**characterized in that** the resetting force is affected by repelling permanent magnets, of which one is arranged on the plunger (22) and the other on the support piece (38) of the housing (10).

7. The device of one of claims 1 to 6,
**characterized in that** the device is a separate component, which is able to be connected with an attachment forming the entrance (12) and with an attachment forming the outlet (18).

8. The device of one of claims 1 to 7,
**characterized in that** the device is integrated in a proximal attachment of a cannula.

## Revendications

1. Dispositif pour limiter la pression d'injection d'un instrument médical pour introduire un fluide comprenant :
* un boîtier (10),
* une entrée (12) du boîtier (10) pour introduire le fluide à une pression d'entrée,
* une sortie (18) du boîtier (10) pour alimenter l'instrument en fluide,
* un canal de passage reliant l'entrée (12) et la sortie (18) dans le boîtier (10),
* un piston (22) guidé dans le boîtier (10) appliquant la pression du fluide dans le canal de passage et qui est déplacé par cette pression contre une force de rappel,
dispositif dans lequel
- le boîtier (10) a une cavité (20) dans laquelle le piston (22) est guidé axialement de manière étanche,
- la force de rappel agit co-axialement à la direction de mouvement du piston d'une part sur le piston (22) et d'autre part sur un embout (38) du boîtier (10),
- le piston (22) comporte un élément de fermeture (30, 66) qui se trouve dans la section de passage du canal de passage,
- l'élément de fermeture (30, 66) libère la section de passage lorsque la pression du fluide appliquée sur le piston (22) est en-dessous d'une valeur prédéfinie par la force de rappel et il ferme la section de passage si la pression du fluide appliqué sur le piston (22) dépasse cette valeur prédéfinie et déplace le piston (22) contre la force de rappel,
- l'entrée (12) et la sortie (18) sont situées sur un axe longitudinal commun et la cavité (20) est réalisée dans le boîtier (10) co-axialement à cet axe longitudinal,
dispositif **caractérisé en ce que**
la sortie (18) est réalisée sous la forme d'un tube intérieur (14) co-axial dans le boîtier (10) qui débouche dans la cavité (20) à son extrémité ouverte située en amont,
le piston (22) est guidé sur ce tube intérieur (14) et l'élément de fermeture est un plateau de soupape (30) fixé co-axiallement au piston (22) et s'appuyant de façon étanche axialement sur l'extrémité ouverte (16) du tube intérieur (14) pour fermer la section de passage et se soulevant axialement de l'extrémité ouverte (16) du tube intérieur (14) pour libérer la section de passage,
- la surface frontale du piston (22) tournée vers l'entrée (12) ayant un embout (28) cylindrique creux fermé par le plateau de soupape (30),
- le plateau de soupape (30) a la forme d'un disque circulaire dont le diamètre dépasse le diamètre extérieur du tube intérieur (14) et la paroi de l'embout (28) est traversée par les orifices de passage (32).

2. Dispositif selon la revendication 1,
**caractérisé en ce que**
la force de rappel est réglable.

3. Dispositif selon les revendications 1 et 2,
**caractérisé en ce que**
l'embout (38) est réglable axialement dans le boîtier (10) pour régler la force de rappel.

4. Dispositif selon l'une des revendications 1 à 3,
**caractérisé en ce que**
la force de rappel est produite par un ressort hélicoïdal de compression (36) co-axial à la direction de mouvement du piston, ce ressort s'appuyant d'un côté contre le piston (22) et de l'autre contre l'embout (38) du boîtier (10).

5. Dispositif selon l'une des revendications 1 à 4,
**caractérisé en ce que**
la force de rappel est produite par un élément (80) comprimable élastiquement dans la direction de mouvement du piston, et qui d'un côté s'appuie contre le piston (22) et de l'autre contre l'embout (38) du boîtier (10).

6. Dispositif selon l'une des revendications 1 à 5,
**caractérisé en ce que**
la force de rappel est produite par des aimants permanents qui se repoussent, dont l'un est porté par le piston (22) et l'autre par l'embout (38) du boîtier (10).

7. Dispositif selon l'une des revendications 1 à 6,
**caractérisé en ce que**
le dispositif est un composant distinct qui peut être relié à un raccord formant l'entrée (12) et à un raccord formant la sortie (18).

8. Dispositif selon l'une des revendications 1 à 7,
**caractérisé en ce que**
le dispositif est intégré dans un épaulement proximal d'une canule.
